Europäisches Patentamt

**European Patent Office**

Office européen des brevets

(19)

(11) Numéro de publication : **0 006 822**
**B1**

(12)

# FASCICULE DE BREVET EUROPÉEN

(45) Date de publication du fascicule du brevet :
13.10.82

(51) Int. Cl.³ : **G 01 N 33/92**

(21) Numéro de dépôt : 79400450.7

(22) Date de dépôt : 02.07.79

(54) **Procédé et ensemble de réactifs pour le dosage du cholestérol sérique.**

(30) Priorité : 04.07.78 GB 28815/78
22.01.79 GB 7902255

(43) Date de publication de la demande :
09.01.80 (Bulletin 80/01)

(45) Mention de la délivrance du brevet :
13.10.82 Bulletin 82/41

(84) Etats contractants désignés :
**BE DE FR GB IT LU NL SE**

(56) Documents cités :
**WO A 79/00306
DE A 2 600 664
US A 3 823 129
US A 3 884 638
US A 3 884 764
US A 4 039 285
US A 4 045 176**
Chemical Abstracts. Volume 84, no 3. 19 janvier 1976, (COLUMBUS, OHIO, USA) A.K. HARMONY et al. « Interaction of human plasma low density lipoprotein with concanavalin A and with ricin » page 161 abrégé no 13784T
Chemical Abstracts. Volume 81, no 15. 14 octobre 1974, (COLUMBUS, OHIO, USA) W.J. McCO-NATHY et al. « Interaction of concanavalin A with major density classes of human plasma lipoproteins. Evidence for the specific binding of lipoprotein B in its associated and free forms » page 136 abrégé no 87328G

(73) Titulaire : **LABORATOIRES GOELLA**
**5, rue des Colonnes du Trône**
**F-75012 Paris (FR)**

(72) Inventeur : **Pascal, Marc**
**5, rue Hernoux**
**F-21000 Dijon (FR)**

(74) Mandataire : **Peaucelle, Chantal et al**
**Cabinet Plasseraud 84, rue d'Amsterdam**
**F-75009 Paris (FR)**

Imprimerie Jouve, 18, rue St-Denis, 75001 Paris, France

### Procédé et ensemble de réactifs pour le dosage du cholestérol sérique

L'invention est relative à des moyens, procédés et ensembles de réactifs pour le dosage de cholestérol sérique.

Le rôle du cholestérol dans les affections cardio-vasculaires est étudié depuis longtemps. Jusqu'à une époque récente cependant, les analyses conduites pour détecter les risques d'apparition de ces affections portaient essentiellement, pour ce qui concerne la lipidémie, sur la détermination du cholestérol total présent dans le sérum.

Cependant, depuis quelques années, dans le domaine des lipides, l'influence du cholestérol total, ou encore des triglycérides, sur l'incidence des maladies cardio-vasculaires a été étudiée de façon plus approfondie du fait de l'existence de cas relativement fréquents remettant en cause la corrélation antérieurement admise entre la teneur en cholestérol total et l'apparition notamment d'infarctus du myocarde.

On sait que le cholestérol se trouve dans le sérum dans des lipoprotéines. Celles-ci ne se présentent pas à l'analyse de façon uniforme, et l'on distingue notamment, en fonction de critères de densité :

— les lipoprotéines de très faible densité, ou VLDL selon les initiales des termes britanniques servant à les désigner $(0,96 < d < 1,000)$,

— les lipoprotéines de faible densité, ou LDL $(1,006 < d < 1,063)$,

— les lipoprotéines de haute densité, ou HDL $(1,063 < d)$.

A ces diverses catégories de lipoprotéines correspondent des apoprotéines particulières désignées par $C_1$, $C_2$, $C_3$, E, B, $A_1$, $A_2$. Certaines de ces apoprotéines sont spécifiques d'une ou de plusieurs catégories de lipoprotéines. Ainsi, l'apoprotéine B est spécifique des lipoprotéines LDL et VLDL.

Les études récentes pour le dépistage du risque d'affections cardio-vasculaires se sont orientées vers des dosages différenciés des diverses catégories de lipoprotéines-cholestérol. De tous les travaux effectués dans ce sens, il ressort que, si le cholestérol total est toujours un paramètre important, il ne permet pas, seul, de déterminer le risque de ces affections. Il semble, en effet, que le facteur essentiel soit le taux de cholestérol HDL. Pour atteindre à la meilleure évaluation possible de ce risque, il semble qu'il soit nécessaire de déterminer les taux de cholestérol HDL, d'une part, et LDL et VLDL, d'autre part.

Une forme commode de représentation du risque de ces maladies cardio-vasculaires est constituée par le rapport du cholestérol de la fraction HDL par rapport à celui des fractions LDL et VLDL.

$$R = \frac{HDL - cholestérol}{(LDL = VLDL)\ cholestérol}$$

Comme nous l'avons dit, les principales méthodes de dosage couramment utilisées dans les laboratoires d'analyses médicales avaient pour but la détermination du taux de cholestérol total. Ces méthodes avaient pour avantage de n'exiger qu'un minimum de matériel et des réactifs relativement simples. Les études dont il a été question ci-dessus ayant montré l'insuffisance de ce type d'analyse, de nouveaux types ont été essayés avec plus ou moins de succès.

Il a été proposé notamment de doser le cholestérol des β-lipoprotéines (LDL) après les avoir isolées par précipitation à l'aide d'héparine en présence de cations divalents. Cette technique de séparation, dans le meilleur cas, a pour inconvénient de ne pas être suffisamment spécifique. Il en résulte une trop grande imprécision, notamment sur les taux de cholestérol HDL, pour qu'il soit permis d'utiliser cette méthode pour l'évaluation des risques, et notamment du facteur R indiqué précédemment.

A l'heure actuelle, les seuls moyens dont on dispose pour effectuer la séparation et le dosage des diverses fractions lipoprotéines-cholestérol dans des conditions de précision satisfaisantes sont basés sur des méthodes d'ultracentrifugation sur un milieu présentant un gradient de densité.

Le matériel nécessaire pour ce type d'opération est très coûteux et n'a pas d'autre usage dans les laboratoires d'analyses médicales.

Par ailleurs, la mise en œuvre de ces séparations de lipoprotéines est relativement longue, ce qui ajoute au coût et limite le nombre d'analyses possibles dans un temps donné.

Pour toutes ces raisons, les dosages de cholestérol, pour déterminer au mieux les facteurs de risques de maladies vasculaires tels qu'ils sont apparus au cours des études récentes, ne sont pratiqués que de façon exceptionnelle alors que leur grand intérêt justifierait un emploi systématique.

Un but de l'invention est donc de fournir des moyens qui permettent la détermination des facteurs de risques d'affections cardio-vasculaires liées aux variations des taux du cholestérol sérique, et qui ne présentent pas les inconvénients des méthodes antérieures.

L'invention a notamment pour but de fournir des moyens de dosage du cholestérol sérique par fraction de lipoprotéines, moyens qui soient accessibles à tout laboratoire d'analyses médicales sans entraîner de couteux investissements. L'invention a aussi pour but des moyens permettant d'aboutir à des dosages dont la durée et le coût soient compatibles avec une mise en œuvre de dépistages ou de contrôles systématiques.

Pour parvenir à de tels moyens, la demanderesse s'est d'abord efforcée de trouver une technique de séparation des lipoprotéines sériques qui soit suffisamment spécifique tout en gardant l'avantage d'une mise en œuvre relativement simple. Après diverses recherches, la demanderesse a découvert qu'une technique répondant à ces exigences consistait en une séparation par précipitation sélective des lipoprotéines LDL et VLDL, par l'addition de lectines aux échantillons de sérum étudiés.

Dans le Chem. Abstr. *81,* page 136, N° 87328q (1974), on rapporte une interaction entre la concanavaline A fixée sur du Sépharose *4* B avec les lipoprotéines B.

L'interaction des lipoprotéines et des lectines a été confirmée par l'étude de l'effet des lipoprotéines sur l'agglutinabilité des érythrocytes induite par les lectines.

La demanderesse a étudié et montré que cette interaction entre lectines et lipoprotéines pouvait conduire à une séparation des fractions HDL, d'une part, et, d'autre part, LDL + VLDL par précipitation de ces dernières, et que cette précipitation pouvait répondre aux conditions de spécificité requises pour permettre un dosage suffisamment précis du cholestérol appartenant à ces fractions. Grâce à cette découverte, la demanderesse a pu atteindre les buts qu'elle s'était fixés.

Le procédé de dosage de cholestérol sérique selon l'invention comprend une séparation des lipoprotéines LDL et VLDL, d'une part, et HDL, d'autre part, puis la détermination de la quantité de cholestérol contenue dans au moins l'une des deux fractions. De préférence, la détermination de la teneur en cholestérol est effectuée sur les deux fractions séparées. Ce procédé est caractérisé en ce que la séparation des deux fractions de lipoprotéines est obtenue par une précipitation sélective des fractions LDL et VLDL au moyen d'une lectine capable de former des complexes insolubles avec ces lipoprotéines.

Les mécanismes chimiques ou physico-chimiques, qui font que les lipoprotéines LDL et VLDL sont précipitées par certaines lectines alors que, dans les mêmes conditions, les lipoprotéines HDL restent solubles, ne sont pas parfaitement élucidés. Les études réalisées notamment par la demanderesse montrent cependant de façon suffisante qu'il est possible d'obtenir avec certaines lectines (encore nommées phytohémagglutinines) une action très spécifique sur les lipoprotéines sériques en fonction de leur densité. Dans ce type de réaction sélective, aboutissant notamment à la formation de complexes insolubles, on peut raisonnablement, compte tenu de l'état des connaissances actuelles, émettre l'hypothèse que ces lectines ont une affinité pour certains sites particuliers présents dans les lipoprotéines en question. Ces sites sont notamment des motifs osidiques.

Compte tenu de ces hypothèses, des lectines préférées sont celles qui manifestent une affinité pour les motifs en question et notamment les résidus galactose.

Quoi qu'il en soit, le choix des lectines utilisées pour la mise en œuvre de l'invention repose essentiellement sur des critères pratiques. Il consistera essentiellement, pour le technicien, à vérifier, parmi les lectines disponibles, celles qui précipitent ces lipoprotéines de faibles densités (LDL et VLDL) sans entraîner la précipitation des lipoprotéines de forte densité.

Parmi les lectines utilisables, la concanavaline (lectine extraite de Canavalia ensiformis) est particulièrement avantageuse pour la mise en œuvre de l'invention, mais d'autres lectines peuvent être utilisées sous la réserve qu'elles présentent les caractères de spécificité indiqués ci-dessus.

Pour conduire à la séparation des lipoprotéines, il va de soi que la quantité de lectine ajoutée doit être suffisante, compte tenu de l'importance de l'échantillon de sérum traité. Les conditions de concentration interviennent également dans les déplacements des équilibres chimiques qui sont à l'origine de la réaction de précipitation de composés complexes.

Ainsi, l'addition d'une lectine à un échantillon de sérum, l'ensemble étant fortement dilué, peut aboutir à une précipitation incomplète des lipoprotéines de faible densité même si, par ailleurs, la quantité de lectine utilisée devrait suffir à précipiter la totalité de ces lipoprotéines.

A l'inverse, en milieu très concentré, la précipitation, qui s'opère par priorité sur les lipoprotéines de faible densité, peut s'étendre à une partie des lipoprotéines de densité élevée.

Il est préférable, pour la simplicité et la reproductibilité du dosage, d'opérer une séparation pratiquement complète des deux types de lipoprotéines que nous avons distingués. On se place donc avantageusement, pour chaque lectine utilisée, dans le domaine de concentration pour lequel la précipitation des lipoprotéines LDL et VLDL est complète et celle des lipoprotéines HDL est pratiquement nulle.

Pour la concanavaline en solution aqueuse, lorsque les échantillons de sérum étudiés n'ont pas subi de dilution préalable, un domaine de concentration avantageux se situe entre environ 0,8 mg/ml et 3 mg/ml. De préférence, on utilise une solution contenant environ 1 mg/ml.

A titre indicatif, pour la concanavaline, des solutions dont la concentration est égale ou supérieure à 10 mg/ml entraînent la précipitation d'une partie non négligeable de lipoprotéines HDL présentes dans le sérum, qui peut atteindre 10 %.

Les réactions envisagées dans la méthode selon l'invention, mettant en jeu le déplacement d'équilibres chimiques, ne sont pas instantanées. Il est donc nécessaire de maintenir le mélange réactionnel jusqu'à ce que la formation du complexe insoluble soit pratiquement complète. A la température ambiante et dans les conditions préférées, après 1 heure, la réaction peut être considérée comme complète.

Pour le traitement de certains échantillons de sérum, particulièrement ceux pour lesquels la teneur en triglycérides est spécialement élevée (plus de 10 g/l, par comparaison avec la teneur considérée comme normale et qui est de 0,5 à 1,5 g/l), il peut se produire une précipitation incomplète de la fraction la plus légère. La proportion de lipoprotéines légères non précipitées est toujours faible, mais, lorsqu'elle se trouve dosée avec les HDL, elle peut entraîner une erreur significative dans l'évaluation de ces dernières, et par suite du facteur de risque.

Pour éviter une telle erreur, on a montré que la précipitation complète des lipoprotéines de faible densité peut être obtenue en ajoutant au mélange dans lequel s'effectue la précipitation une quantité suffisante d'un polymère hydrosoluble, neutre, polaire, et qui n'a pas de propriétés tensioactives. De tels polymères modifient la polarité de la solution, ce qui accroît l'insolubilité de ces lipoprotéines. Des polymères préférés, qui peuvent être utilisés à cette fin, sont notamment les polymères de polyéthylèneglycol ou de polypropylène-glycol. De façon particulièrement préférée, on utilise des polymères de polyéthylèneglycol dont le poids moléculaire moyen est de 200 à 20 000. De tels polymères sont avantageusement ajoutés en quantités telles que leur concentration dans le mélange de précipitation est de 0,5 à 5 % en poids.

Un autre avantage lié à l'utilisation de ces polymères est que le temps nécessaire pour atteindre une précipitation complète est considérablement diminué.

L'utilisation de ces polymères est avantageuse pour tous les procédés dans lesquels une séparation des lipoprotéines est effectuée par précipitation des fractions de faible et très faible densité (LDL et VLDL). Ces polymères sont particulièrement efficaces dans la précipitation au moyen de lectines ; néanmoins, ils sont également utiles pour la précipitation au moyen de systèmes tels que les polyanions (héparine et ions métalliques par exemple). Dans tous les cas, la précipitation des protéines les plus légères est améliorée.

La précipitation étant effectuée, la séparation du complexe lectine-lipoprotéine (LDL et VLDL) du mélange réactionnel contenant notamment les lipoprotéines HDL est ensuite effectuée de façon et par des moyens traditionnels de séparation d'un précipité. Pour la commodité de l'opération, étant donné la finesse des particules en suspension dans le mélange, il est avantageux d'effectuer une centrifugation, puis de séparer le liquide du résidu solide qui adhère au tube. Une centrifugation modérée ou moyenne, telle qu'elle peut être obtenue avec des dispositifs usuels de laboratoire, suffit pour séparer les particules en suspension. Par exemple, une centrifugation à 1 800 G pendant 10 minutes permet une séparation complète.

Dans tous les cas, cette séparation n'a bien évidemment rien de commun avec celles qui sont effectuées pour séparer directement les protéines en fonction de leur densité. Dans ces dernières, en effet, il est nécessaire d'opérer dans un milieu présentant un gradient de densité et de soumettre l'échantillon à une centrifugation très poussée (ultracentrifugation) nécessitant un matériel très particulier.

Une fois les lipoprotéines séparées comme il vient d'être indiqué, chaque fraction peut être analysée suivant des méthodes connues pour le dosage du cholestérol sérique, en particulier par les méthodes colorimétriques, chimiques ou biochimiques telles que celles dans lesquelles une ou plusieurs réactions enzymatiques sont réalisées.

Il est nécessaire, de toute façon, d'analyser les constituants séparés (précipité et surnageant) dans des conditions telles que la lectine utilisée, la concanavaline dans le mode préféré, et, le cas échéant, les constituants sériques autres que les lipoprotéines liées au cholestérol ne perturbent pas le dosage du cholestérol.

Selon le procédé choisi pour le dosage, la mise en condition des fractions de lipoprotéines peut varier de façon importante. Deux procédés particulièrement avantageux sont décrits en détail ci-après : un procédé dans lequel le cholestérol est dosé après avoir été séparé des constituants sériques qui pourraient interférer dans la mesure ; un procédé dans lequel le cholestérol est soumis à une réaction enzymatique et les composés résultants dosés.

I. Séparation et dosage du cholestérol

Avantageusement, le cholestérol des fractions lipoprotéiniques encore en solution dans le surnageant est isolé par précipitation avant de procéder à son dosage.

Selon l'invention, il est avantageux de procéder à une dénaturation des protéines présentes dans les deux fractions séparées, dénaturation qui s'étend bien entendu à la lectine ayant servi à la précipitation sélective. Dans le cas du surnageant, la dénaturation des protéines entraîne leur précipitation et, avec elle, celle du cholestérol qui leur est lié.

Pour effectuer cette dénaturation, on utilise des moyens traditionnels de la chimie des protéines. En particulier, il est avantageux de dénaturer les protéines, sans risque d'altérer les éléments lipidiques, au moyen de solutions acides. Parmi les acides utilisables, on préfère l'acide trichloracétique (à concentration supérieure à 5 %, et avantageusement de l'ordre de 10 %), connu pour son action dénaturante sur les protéines, les globulines, l'albumine, etc.

Après une dénaturation du genre qui vient d'être dit, on dispose de deux fractions solides précipitées.

Avantageusement également, les lipoprotéines du surnageant provenant de la précipitation fractionnée peuvent être isolées de résidus aqueux sériques par extraction à l'aide de solvants non miscibles à l'eau, utilisés ordinairement dans les techniques de délipidation. Les lipoprotéines extraites peuvent ensuite être isolées en chassant le solvant.

En plus des protéines, d'autres constituants sériques sont susceptibles d'interférer dans les méthodes de dosage du cholestérol, notamment lorsqu'il s'agit de dosages colorimétriques.

Ainsi, sur les résidus récupérés après dénaturation des protéines ou extraction par solvant et qui contiennent le cholestérol, il est préférable d'éliminer les substances chromogènes non cholestériques encore présentes, et notamment la bilirubine ou des dérivés de l'urée.

Suivant un mode avantageux selon l'invention, on opère une solubilisation sélective du cholestérol qui laisse les substances chromogènes à l'état insoluble.

La solubilisation du cholestérol peut être obtenue au moyen de solvants comme l'acide acétique glacial. Il est avantageux selon l'invention, pour améliorer la spécificité de la solubilisation et la sensibilité de la méthode, ou, autrement dit, pour écarter de la solution les substances susceptibles de gêner le dosage ultérieur, comme proposé par BHANDARU R.R. et col. (Lipids, 12, 1 078 (1977)) d'ajouter à l'acide acétique de l'acétate d'uranyle, connu pour être un excellent fixateur de protéine. L'acide acétique seul, en effet, aurait tendance à entraîner la solubilisation des protéines. De façon particulièrement préférée, on utilise une solution contenant environ 0,2 mg/ml d'acétate d'uranyle dans l'acide acétique glacial.

A partir des solutions de cholestérol correspondant aux deux fractions séparées initialement, puis débarrassées des substances qui pourraient rendre certaines méthodes de dosage difficiles ou insuffisamment précises, on peut opérer une analyse quantitative de façon traditionnelle.

Pour des raisons de commodité, on préfère habituellement doser le cholestérol par une mesure colorimétrique. Parmi les diverses méthodes connues pour ce dosage, on préfère celle décrite par ZLATKIS et ZAK. (Anal. Biochem., 29, 143 (1969)) qui permet une manipulation rapide et hautement reproductible. En outre, cette méthode a l'avantage de pouvoir être appliquée sur des quantités très faibles d'échantillons, de l'ordre ou inférieures à 20 µg et même aussi faibles que 5 µg.

Pour cette méthode, on utilise avantageusement comme réactif l'orthophthaldialdéhyde en solution dans un mélange acide acétique-acide sulfhydrique.

II. Traitement enzymatique et dosage

Diverses méthodes pour le dosage du cholestérol sont connues, qui comprennent des réactions enzymatiques. Ces méthodes peuvent être utilisées pour déterminer la teneur en cholestérol dans les deux fractions de lipoprotéines (HDL d'une part, LDL et VLDL d'autre part) séparées par le procédé selon l'invention. Parmi ces méthodes sont particulièrement avantageuses celles décrites par TRINDER P. (J. Clin. Path, 22, 246, 1969) et par RÖSCHLAU P. et al (Z. Klin. Chem. Klin. Biochem., 12, 403, 1974). Dans ces deux méthodes, le principe est le suivant :

— hydrolyse du cholestérol au moyen de cholestérol-estérase ;

— oxydation du cholestérol au moyen de cholestérol-oxydase, conduisant à la cholesténone et à $H_2O_2$ ;

— dosage de $H_2O_2$ par spectrophotométrie.

Quelle que soit la méthode enzymatique choisie, les fractions de lipoprotéines récupérées après l'étape de précipitation doivent être mises dans des conditions permettant la mise en œuvre du traitement enzymatique.

Le précipité obtenu est dissous. Ceci est avantageusement réalisé au moyen d'une solution glucosidique, notamment par une solution d'α-méthyl-glucopyranoside. D'autres glucides peuvent être utilisés tels que le mannose, le galactose, le glucose, etc. En plus de la dissolution des lipoprotéines précipitées, le glucoside permet d'éviter la formation d'éventuelles interactions entre la lectine et d'autres glycoprotéines utilisées dans la suite du dosage, par exemple avec la peroxydase.

Un agent tensio-actif est également avantageux pour maintenir le cholestérol en solution. Un agent tensio-actif préféré est celui commercialisé sous le nom de Triton X 100, mais d'autres agents sont bien entendu utilisables, notamment l'hydroxypolyéthoxydodécane.

Le réglage de la force ionique de la solution est également avantageux. Ceci peut être obtenu de façon classique par addition d'une solution saline, par exemple de chlorure de sodium, de chlorure de potassium ou en utilisant un tampon approprié.

D'autres composés utiles pour les réactions des étapes ultérieures peuvent être ajoutés aux précédents, par exemple du méthanol qui est utile pour la mise en œuvre de la réaction enzymatique.

Lorsque les fractions de lipoprotéines sont sous forme de ces solutions ainsi préparées, le processus habituel des réactions enzymatiques pour le dosage du cholestérol peut être suivi.

Selon l'invention, quelle que soit la méthode de dosage utilisée après la séparation des fractions de lipoprotéines, on aboutit à une analyse quantitative sur au moins l'une de ces fractions et de préférence sur les deux. Il est alors aisé, pour déterminer le risque d'accident vasculaire et en particulier le risque d'athérosclérose, d'établir le rapport cholestérol (LDL + VLDL)/cholestérol HDL et de le comparer aux valeurs statistiques pour les sujets normaux et les sujets atteints de ces affections.

Tout en opérant la séparation prescrite selon l'invention, il est également possible de ne doser qu'une seule des fractions isolées et de combiner cette mesure, par exemple, avec une méthode traditionnelle de dosage du cholestérol total, pour aboutir, par différence, à la valeur manquante. Il est cependant préférable d'effectuer les mesures sur les deux fractions isolées et par la même méthode, ce qui tend à limiter l'influence des erreurs systématiques sur le résultat global.

Pour la mise en œuvre du procédé de dosage du cholestérol selon l'invention, le matériel nécessaire est relativement simple et ne va pas au-delà de l'équipement habituel des laboratoires d'analyses médicales ordinaires dans lesquels sont réalisées les analyses hématologiques. En particulier, il suffit, pour réaliser la séparation, d'utiliser une centrifugeuse de faible puissance, et, pour le dosage proprement dit, par exemple un spectrocolorimètre traditionnel est tout à fait approprié.

Les réactifs nécessaires à la pratique du procédé selon l'invention, et en particulier les lectines, ne figurent pas toujours au nombre des produits usuels dans les laboratoires travaillant sur les analyses de routine les plus simples. Aussi, pour en faciliter l'exécution, l'invention concerne aussi une présentation de réactifs (kit) utiles pour la mise en œuvre du procédé selon l'invention. La présentation selon l'invention est d'autant plus nécessaire que, pour aboutir à des résultats fiables et reproductibles, la qualité des réactifs doit être constante d'une mesure à une autre.

Le réactif le plus sujet à des modifications de qualité susceptibles de compromettre le dosage est la lectine. Laissées à la lumière ou au contact de l'atmosphère du laboratoire sans précautions particulières, les lectines peuvent se dégrader, en particulier lorsqu'elles sont en solution. Pour garantir une bonne conservation de la lectine, il est préférable de la présenter sous une forme lyophilisée et préservée de l'humidité. Il est également préférable de la maintenir à une température inférieure à la température ambiante et de l'ordre de 4 °C.

Pour faciliter la préparation de la solution de lectine au moment du dosage, elle est également présentée de préférence sous forme de quantités dosées correspondant à un volume défini d'eau pour aboutir à une solution à teneur déterminée en lectine correspondant aux valeurs définies plus haut, et de préférence à 1 mg/ml.

D'autres réactifs peuvent être placés dans les présentations « kits » pour la mise en œuvre du procédé selon l'invention. Leur nature dépend de la méthode de dosage envisagée.

A. Pour une réaction de dosage colorimétrique non enzymatique, une présentation des réactifs selon l'invention contient en outre avantageusement de l'acétate d'uranyle, de préférence sous forme de solution prédosée dans l'acide acétique glacial. De façon particulièrement préférée, la solution d'acétate d'uranyle est à 0,2 mg/ml dans l'acide acétique.

Une présentation selon l'invention contient encore avantageusement un réactif de coloration permettant de mettre en évidence et de doser le cholestérol présent par une méthode colorimétrique. De préférence, ce réactif est constitué par l'orthophtaldialdéhyde. Pour ce dernier, il est avantageux de le présenter sous forme prédosée pour la préparation extemporanée de solution dans de l'acide acétique glacial. Un prédosage préféré correspond à des solutions à 1 mg/ml d'orthophtaldialdéhyde dans l'acide acétique glacial.

Avantageusement, dans une présentation selon l'invention figurent aussi une ou des solutions d'étalonnage contenant du cholestérol dans un milieu correspondant, qualitativement et quantitativement, à celui dans lequel les échantillons analysés se trouvent après les opérations effectuées selon l'invention, préalablement au dosage proprement dit.

Suivant les conditions préférées, le milieu des solutions étalons est constitué par une solution d'acétate d'uranyle à 0,2 mg/ml dans l'acide acétique glacial.

Une solution étalon avantageuse, pour une comparaison commode avec les échantillons analysés, contient une quantité bien précise de cholestérol, d'environ 20 µg/ml, dans une solution d'acétate d'uranyle dans l'acide acétique glacial (0,2 mg d'acétate d'uranyle par millilitre d'acide).

D'autres réactifs utiles pour la mise en œuvre du procédé selon l'invention peuvent également figurer dans la présentation. Cependant, s'agissant de produits qui se trouvent communément dans les laboratoires d'analyse, on comprendra que leur présence n'apporte, comme avantage supplémentaire, que celui de fournir dans un même ensemble la totalité des composés nécessaires. Parmi les produits en question figurent en particulier les acides ou solutions acides utiles pour la dénaturation des protéines, en particulier, suivant un mode préféré, les solutions d'acide trichloracétique à concentration égale ou supérieure à 5 %. Figurent aussi les produits annexes pour la préparation de réactifs de façon extemporanée, et en particulier, lorsqu'on utilise comme réactif de coloration l'orthophtaldialdéhyde, l'acide acétique glacial et les solutions d'acide sulfhydrique concentrées.

B. Pour un dosage comprenant une réaction enzymatique selon l'invention, pour effectuer un dosage enzymatique, un kit contient avantageusement des solutions renfermant séparément un glucoside tel que l'α-méthyl-glucopyranoside, un agent tensio-actif, du chlorure de sodium, les enzymes nécessaires, etc.

La présentation des différents constituants nécessaires pour ces dosages peut se faire dans autant de récipients distincts qu'il y a de produits. Pour faciliter le conditionnement comme la mise en œuvre, il est cependant préférable de regrouper autant que possible tous les réactifs dont le mélange est compatible avec une bonne conservation. On peut notamment rassembler, d'une part, les constituants enzymatiques et, d'autre part, le reste des réactifs. On peut également à certaines conditions, notamment sous forme lyophilisée, rassembler tous les réactifs, y compris les enzymes, dans un seul récipient.

Les essais et exemples suivants illustreront de façon plus détaillée les caractéristiques de la présente invention.

1) Essai de spécificité de la méthode de séparation des lipoprotéines par formation de complexes avec la concanavaline A

Dans ces essais, on a utilisé la concanavaline A commercialisée par la Société PHARMA-INDUSTRIE.

La précipitation des lipoprotéines du sérum humain par la concanavaline A a été étudiée sur trois matériaux. Le premier (fraction A) est constitué par du sérum humain complet. Les deuxième et troisième matériaux sont constitués par des fractions obtenues à partir de ce même sérum et isolées par la méthode décrite par Havel et col. dans J. Clin. Invest. *34*, 1345 (1955).

Selon cette méthode, on a séparé et isolé par centrifugation une fraction de densité comprise entre 1,107 et 1,22 (fraction B) et une fraction de densité inférieure à 1,063 (fraction C).

L'analyse par électrophorèse sur gel de polyacrylamide des fractions B et C montre qu'elles contiennent exclusivement, pour la première, des lipoprotéines dites HDL et, pour la seconde, des lipoprotéines LDL et VLDL.

Sur ces fractions, la capacité de précipitation de la concanavaline A a été déterminée pour différentes concentrations et pour des échantillons de sérum plus ou moins dilués.

La précipitation est mesurée par densité optique à la longueur d'onde de 450 nm. A cette longueur d'onde, ni les différents constituants sériques, ni la concanavaline A n'adsorbent.

Une fois faite l'addition de concanavaline A aux différentes fractions, le précipité, lorsqu'il y en a un, et le surnageant sont séparés et analysés par électrophorèse sur gel de polyacrylamide comme précédemment.

Les résultats obtenus sont les suivants :

a) L'apparition du précipité dépend principalement des fractions considérées mais aussi, dans une certaine mesure, des conditions de concentration dans lesquelles les essais sont réalisés.

Pour une solution de concanavaline A à 1 mg/ml, qui s'est révélée être un réactif bien approprié au type de séparation recherché, on observe une précipitation uniquement sur les fractions A et C. L'analyse, dans ces conditions, ne permet pas de déceler de formation de précipité sur la fraction B.

b) Toujours en utilisant la solution de concanavaline A à 1 mg/ml, on constate que la quantité de précipité formée est fonction de la quantité de solution ajoutée. Pour le sérum non dilué ou pour des dilutions allant jusqu'à 1/25, la relation précipité/quantité de solution concanavaline A est pratiquement linéaire.

c) L'analyse de la fraction surnageant après achèvement de la précipitation montre, en particulier pour la fraction C, l'absence complète de lipoprotéines de faible densité. Dans les conditions indiquées, la concanavaline A permet, par conséquent, une précipitation pratiquement complète de ces lipoprotéines.

Une comparaison entre les essais effectués sur les fractions A et C, c'est-à-dire entre celles correspondant au sérum complet et celles ne contenant que les lipoprotéines LDL et VLDL, montre que l'addition de concanavaline A entraîne la précipitation de ces lipoprotéines, mais également celle d'autres protéines sériques.

Il n'est donc pas possible, à partir d'un échantillon de sérum complet, d'envisager une méthode de mesure, par exemple par densité optique à 450 nm, aboutissant directement à la détermination des teneurs en lipoprotéines LDL et VLDL.

d) Toujours dans les conditions indiquées, c'est-à-dire pour une précipitation conduite à l'aide d'une solution 1 mg/ml, on s'est assuré que, dans le précipité obtenu à partir du sérum complet, les lipoprotéines HDL n'étaient pas coprécipitées même partiellement (ceci en plus du fait qu'on ne constate pas de précipité avec la fraction B). Pour cela, on redissout le précipité obtenu à l'aide d'une solution d'$\alpha$-D-méthyl-glucopyranoside 0,2 M. L'affinité de la lectine pour les résidus saccharidiques des lipoprotéines est alors masquée par la présence de ces sucres additionnels et le complexe précipité se redissout. L'électrophorèse de la solution ainsi obtenue, pour ce qui concerne les lipoprotéines permet d'établir la présence des seules lipoprotéines B, c'est-à-dire des fractions LDL et VLDL.

e) Lorsque la concentration de la solution de concanavaline A est réduite par rapport à la solution préférée indiquée ci-dessus, les observations précédentes se retrouvent à l'exception de celles relatives au fait que la précipitation des lipoprotéines LDL et VLDL soit totale. Si la concentration est trop faible, en effet, une partie de ces lipoprotéines peut rester en solution dans le surnageant avec les lipoprotéines HDL.

De la même façon, lorsque la concentration en concanavaline A dépasse un certain seuil, on a tendance à précipiter non seulement les lipoprotéines de basse densité, mais aussi une partie, croissant avec la concentration, des lipoprotéines HDL.

Dans la pratique, pour éviter une précipitation sensible du complexe avec les HDL, il est préférable de maintenir la solution à moins de 10 mg/ml.

Que l'on ne précipite pas la totalité de VLDL et LDL ou que l'on précipite une partie des HDL, le dosage reste réalisable dès lors que les conditions sont reproduites de façon constante et que l'on peut déterminer préalablement un facteur de correction. Il est cependant préférable de rester dans le domaine pour lequel à la fois la précipitation des LDL et VLDL est pratiquement complète, et d'autre part les HDL restent en solution.

Les résultats précédents montrent la spécificité de la méthode de séparation des lipoprotéines. On s'est encore assuré que les taux de cholestérol des fractions isolées de cette façon correspondaient bien à ceux qui peuvent être déterminés après une séparation faite par ultracentrifugation. Autrement dit, on a vérifié que la méthode de séparation des lipoprotéines proposée est compatible avec un dosage du cholestérol.

On a en particulier montré que la somme des teneurs en cholestérol de chaque fraction isolée en suivant le protocole de l'invention décrit ci-après au 2) correspondait bien à la valeur trouvée pour le cholestérol total en appliquant une méthode traditionnelle de dosage.

2) Exemple de mise en œuvre du procédé selon l'invention

Pour conduire le dosage du cholestérol des fractions LDL et VLDL, et HDL, les réactifs suivants ont été utilisés :

— réactif A : solution à 1 mg/ml de concanavaline A dans du sérum physiologique à préparer extratemporanément,

— réactif B : solution aqueuse à 10 % d'acide trichloracétique,

— réactif C : solution à 0,2 mg/ml d'acétate d'uranyle dans l'acide acétique glacial,

— réactif D : solution à 1 mg/ml d'orthophtaldialdéhyde dans l'acide acétique glacial, à préparer extratemporanément et à conserver à l'abri de la lumière,

— réactif E : acide sulfhydrique concentré, solution étalon : solution à 20 µg/ml de cholestérol dans le réactif C.

Le procédé comprend les étapes suivantes : précipitation sélective et séparation des fractions lipoprotéiniques, élimination des constituants indésirables de chaque fraction isolée, et dosage du cholestérol dans chacune des fractions.

a) Séparation des fractions

Dans un tube à hémolyse (tube I), on ajoute 0,03 ml de sérum humain à 1 ml de réactif A. Après agitation, on laisse reposer 1 heure à température ambiante pour que la réaction soit complète.

Le mélange est ensuite soumis à une centrifugation à 1 800 G pendant 10 minutes pour séparer le précipité du surnageant.

0,7 ml de surnageant sont prélevés et placés dans un autre tube à hémolyse (tube II). Le reste de surnageant est éliminé en renversant le tube I et en tapotant celui-ci pour éliminer les gouttes adhérant aux parois. Le précipité reste collé au fond du tube I.

b) Elimination des constituants susceptibles d'interférer lors du dosage.

Dans chacun des tubes I et II, on ajoute 0,7 ml du réactif B dans le but, notamment, de dénaturer la concanavaline présente en excès. Les tubes sont agités pour bien mélanger les constituants, puis centrifugés à 1 800 G pendant 5 minutes.

Les surnageants sont éliminés en renversant les deux tubes, comme précédemment.

Dans chaque tube, on ajoute 2 ml de réactif C, et on agite vigoureusement le mélange qui est ensuite laissé au repos pendant 30 minutes, puis centrifugé 20 minutes à 3 000 G.

Dans les tubes I et II, on prélève respectivement 0,4 ml et 1 ml que l'on place dans des tubes à essais I' et II'.

c) Dosage colorimétrique du cholestérol dans chaque fraction et comparaison à un échantillon étalon et à une solution dépourvue de cholestérol et traitée de façon équivalente (blanc).

On ajuste le volume du tube I' à 1 ml en ajoutant 0,6 ml de réactif C. On introduit également 1 ml de ce réactif dans le tube pour essai à blanc, et, de même, 1 ml de la solution étalon dans le quatrième tube.

A chacun des quatre tubes ainsi préparés, on ajoute 1 ml du réactif D. On laisse reposer 15 minutes à la température ambiante, puis on ajoute à chaque tube 1 ml de réactif E. On agite vigoureusement et on laisse reposer 10 minutes avant de procéder à la mesure.

Les solutions, préparées comme il vient d'être indiqué, sont passées dans l'heure suivante dans un spectrocolorimètre. Les mesures sont faites à 560 nm par rapport au tube d'essai à blanc pour les tubes I', II' et le tube contenant la solution étalon.

Les taux de cholestérol des deux fractions séparées, en fonction des densités optiques mesurées, s'expriment de la façon suivante

$$\text{cholestérol HDL} = \frac{\text{densité optique tube II'}}{\text{densité optique étalon}} \times 190,5 \text{ en mg/100 ml}$$

$$\text{cholestérol LDL + VLDL} = \frac{\text{densité optique tube I'}}{\text{densité optique étalon}} \times 333,3 \text{ en mg/100 ml}$$

A partir de ces résultats ou directement, on peut calculer le facteur de risque défini plus haut :

$$R = \frac{\text{cholestérol HDL}}{\text{cholestérol LDL + VLDL}} = \frac{\text{densité optique tube II'}}{\text{densité optique tube I'}} \times 0,572$$

Bien entendu, on peut aussi déterminer le taux de cholestérol total.

La méthode ainsi décrite nécessite, comme on l'a vu, un minimum de matériel et, par rapport aux techniques actuelles faisant appel à l'ultracentrifugation, elle est beaucoup plus rapide. Un opérateur utilisant cette méthode de façon routinière peut réaliser sans difficulté au moins une cinquantaine de dosages quotidiennement.

En outre, la méthode conduit à des résultats d'une précision au moins aussi bonne que celle à laquelle aboutissaient les techniques antérieures telles que celle mettant en œuvre une ultracentrifugation et présente l'avantage supplémentaire, par rapport à ces dernières, de ne demander qu'un volume très limité d'échantillon de sérum (30 µl au lieu de 5 ml environ).

**Revendications**

1. Procédé de dosage du cholestérol sérique comprenant une séparation des fractions de lipoprotéines selon leur densité, puis la détermination de la teneur en cholestérol d'au moins l'une de ces fractions, caractérisé en ce que la séparation est effectuée, à partir d'un échantillon sérique, par précipitation sélective des fractions LDL et VLDL (renfermant l'apoprotéine B) au moyen d'une lectine capable de former des complexes insolubles avec ces liproprotéines.

2. Procédé de dosage selon la revendication 1, caractérisé en ce que la lectine utilisée est la concanavaline.

3. Procédé de dosage selon l'une quelconque des revendications précédentes, caractérisé en ce que l'on utilise une quantité et une concentration de lectine telles que la totalité des lipoprotéines LDL et VLDL soit précipitée, cette concentration restant cependant inférieure à celle au-delà de laquelle une partie des lipoprotéines HDL pourrait être coprécipitée.

4. Procédé de dosage selon la revendication 2, caractérisé en ce que, lorsqu'on utilise la concanavaline, sa concentration dans le milieu où la précipitation se déroule est comprise entre 0,8 mg/ml et 3 mg/ml.

5. Procédé de dosage selon l'une quelconque des revendications précédentes, caractérisé en ce que, pour améliorer la précipitation, on ajoute, dans le milieu de précipitation, un polymère neutre, polaire, non tensio-actif.

6. Procédé de dosage selon la revendication 5, caractérisé en ce que le polymère utilisé est un polyéthylèneglycol ou un polypropylèneglycol dont le poids moléculaire moyen est compris entre 200 et 20 000.

7. Procédé de dosage selon la revendication 6, caractérisé en ce que la teneur en polymère du milieu dans lequel s'effectue la précipitation est comprise entre 0,5 et 5 % en poids.

8. Procédé selon l'une quelconque des revendications précédentes, caractérisé en ce que, avant la mesure de la teneur en cholestérol contenu séparément, soit dans le précipité, soit dans le liquide surnageant, on effectue une dénaturation des protéines.

9. Procédé selon la revendication 8, caractérisé en ce que, à partir des précipités de lipoprotéines LDL et VLDL, d'une part, ou de HDL résultant de la dénaturation, d'autre part, on élimine les constituants sériques susceptibles d'interférer avec le cholestérol lors de la mesure de la teneur de ce dernier, cette élimination étant réalisée par resolubilisation sélective du cholestérol.

10. Procédé selon la revendication 9, caractérisé en ce que la solubilisation sélective est effectuée au moyen d'une solution d'acétate d'uranyle dans l'acide acétique glacial.

11. Procédé selon l'une quelconque des revendications précédentes, caractérisé en ce que la ou les fractions de cholestérol isolées sont dosées par colorimétrie, le réactif colorant utilisé pour la colorimétrie étant l'orthophtaldialdéhyde en solution dans un mélange acide acétique glacial-acide sulfhydrique.

12. Procédé de dosage selon l'une quelconque des revendications 1 à 7, caractérisé en ce que la teneur en cholestérol dans les fractions séparées de lipoprotéines est mesurée par des techniques mettant en jeu des réactions enzymatiques.

13. Présentation « kit » pour la mise en œuvre du procédé selon l'une quelconque des revendications précédentes, caractérisé en ce qu'elle comprend, dans des conditionnements distincts, de la concanavaline sous forme de quantités prédosées, de l'acétate d'uranyle et de l'orthophtaldialdéhyde.

14. Présentation selon la revendication 13, caractérisée en ce qu'elle contient une solution de cholestérol étalonnée.

## Claims

1. Method of measuring serum cholesterol comprising separation of the fractions of lipoproteins according to their volumic mass and then determination of the cholesterol content of at least one of these fractions, characterised in that the separation is carried out from a serum sample by selective precipitation of the LDL and VLDL fractions containing the apoprotein B by means of a lectin capable of forming unsoluble complexes with both lipoproteins.

2. Method of measurement according to claim 1, characterised in that the lectin used is concanavalin.

3. Method of measurement according to claim 1, characterised in that an amount and a concentration of lectin are used such that all the LDL and VLDL lipoproteins are precipitated, this concentration, however, remaining lower than that beyond which part of the HDL lipoproteins could be precipitated at the same time.

4. Method of measurement according to claim 2, characterised in that when concanavalin is used its concentration in the medium in which the precipitation takes place is between 0.8 mg/ml and 3 mg/ml.

5. Method of measurement according to anyone of the preceding claims, characterized in that a neutral, polar and non-tensioactive polymer is added to the precipitation mixture to impose the precipitation.

6. Method of measurement according to claim 5, characterised in that the polymer is a polyethyleneglycol or a polypropyleneglycol polymer, the average molecular weight of which is from 200 to 20 000.

7. Method of measurement according to claim 6, characterised in that the polymer content in the precipitation mixture is from 0.5 to 5 w.%.

8. Method according to anyone of the preceding claims, characterised in that before measurement of the cholesterol content either in the precipitate, or in the supernatant liquid, or in both separately, denaturation of the proteins is effected.

9. Method according to claim 8, characterised in that the serum constituents liable to interfere with the cholesterol on measurement of the content of the latter are eliminated from the precipitates of LDL and VLDL lipoproteins, on the one hand, or of HDLs resulting from the denaturation, on the other hand, this elimination being obtained by selective resolubilisation of the cholesterol.

10. Method according to claim 9, characterised in that the selective solubilisation is effected by means of a solution of uranyl acetate in glacial acetic acid.

11. Method according to any one of the preceding claims,. characterised in that the isolated cholesterol fraction or fractions is or are measured by colorimetry, the colouring reagent used being orthophthaldialdehyde in solution in a mixture of glacial acetic acid and sulphydric acid.

12. Method of measurement according to any one of claims 1 to 7, characterised in that the cholesterol content of the separated fractions of lipoproteins is determined by methods including enzymatic reactions.

13. Kit for carrying out the method according to any one of the preceding claims, characterised in that it contains different packings, premeasured amounts of concanavaline, uranyl acetate and orthophthaldialdehyde.

14. Kit according to claim 13, characterised in that it contains a standardized cholesterol solution.

**Ansprüche**

1. Verfahren zur Bestimmung von Cholesterin in Serum, durch Trennung von Lipoproteinfraktionen nach ihrer Dichte und anschließende Bestimmung des Cholesteringehalts von mindestens einer dieser Fraktionen, dadurch gekennzeichnet, daß man die Trennung ausgehend von einer Serumprobe durch selektive Ausfällung der Fraktionen LDL und VLDL (die Apoprotein B enthalten) mittels eines Lectins bewirkt, das geeignet ist zur Bildung von unlöslichen Komplexen mit diesen Lipoproteinen.

2. Verfahren zur Bestimmung nach Anspruch 1, dadurch gekennzeichnet, daß das verwendete Lectin Concanavalin ist.

3. Verfahren zur Bestimmung nach einem der vorhergehenden Ansprüche, dadurch gekennzeichnet, daß man eine derartige Menge und Konzentration an Lectin verwendet, daß die Gesamtheit der Lipoproteine LDL und VLDl ausgefällt wird, wobei diese Konzentration jedoch unter der bleibt, von der an ein Teil der Lipoproteine HDL mitausgefällt werden könnte.

4. Verfahren zur Bestimmung nach Anspruch 2, dadurch gekennzeichnet, daß man bei der Verwendung von Concanavalin dessen Konzentration in dem Medium, in dem die Ausfällung erfolgt, zwischen 0,8 mg/ml und 3 mg/ml liegt.

5. Verfahren zur Bestimmung gemäß einem der vorhergehenden Ansprüche, dadurch gekennzeichnet, daß man zur Verbesserung der Ausfällung in das Ausfällungsmedium ein neutrales, polares, nicht-oberflächenaktives Polymeres fügt.

6. Verfahren zur Bestimmung nach Anspruch 5, dadurch gekennzeichnet, daß das verwendete Polymere ein Polyäthylenglykol oder ein Polypropylenglykol ist, dessen mittleres Molekulargewicht bei 200 bis 20 000 liegt.

7. Verfahren zur Bestimmung nach Anspruch 6, dadurch gekennzeichnet, daß der Gehalt an Polymerem in dem Medium, in dem die Ausfällung vollzogen wird, zwischen 0,5 und 5 Gew.-% liegt.

8. Verfahren nach einem der vorhergehenden Ansprüche, dadurch gekennzeichnet, daß man vor der Messung des Cholesteringehalts, der getrennt entweder in der Ausfällung oder in der überstehenden Flüssigkeit enthalten ist, eine Denaturierung der Proteine bewirkt.

9. Verfahren nach Anspruch 8, dadurch gekennzeichnet, daß man auf der Grundlage der Ausfällungen von Lipoproteinen LDL und VLDL einerseits oder HDL, resultierend aus der Denaturierung, andererseits, die Serumbestandteile, die mit dem Cholesterin bei der Messung von dessen Gehalt, interferrieren können, entfernt, wobei diese Entfernung durch selektives Wieder-Löslichmachen des Cholesterins bewirkt wird.

10. Verfahren nach Anspruch 9, dadurch gekennzeichnet, daß das selektive Löslichmachen mittels einer Uranylacetatlösung in Eisessig bewirkt wird.

11. Verfahren nach einem der vorhergehenden Ansprüche, dadurch gekennzeichnet, daß man die isolierte Fraktion oder die isolierten Fraktionen von Cholesterin durch Colorimetrie bestimmt, wobei das Farbreagens, das für die Colorimetrie verwendet wird, Orthophthaldialdehyd, gelöst in einem Gemisch von Eisessig-Schwefelwasserstoff ist.

12. Verfahren zur Bestimmung nach einem der Ansprüche 1 bis 7, dadurch gekennzeichnet, daß der Gehalt an Cholesterin in den abgetrennten Fraktionen von Lipoproteinen durch Techniken gemessen wird, die sich enzymatischer Reaktionen bedienen.

13. « Kit »-Ausführungsform zur Durchführung des Verfahrens nach einem der vorhergehenden Ansprüche, dadurch gekennzeichnet, daß sie in getrennten Verpackungen Concanavalin in der Form vorbestimmter Mengen, Uranylacetat und Orthophthaldialdehyd enthält.

14. Ausführungsform nach Anspruch 13, dadurch gekennzeichnet, daß sie eine geeichte Lösung von Cholesterin enthält.